# EUROPEAN PATENT SPECIFICATION

(11) **EP 3 222 283 B1**
(45) Date of publication and mention of the grant of the patent: **22.07.2020**
(21) Application number: 17161047.0
(22) Date of filing: 15.03.2017
(51) Int. Cl.: A61K 36/07, A61K 36/074, A61K 31/593, A61K 31/519, A61K 31/385, A61K 31/202, A61P 15/00

(54) **LENTINAN FOR USE IN THE TREATMENT OF ENDOMETRIOSIS**
LENTINAN ZUR VERWENDUNG IN DER BEHANDLUNG VON ENDOMETRIOSE
LENTINAN POUR UTILISATION DANS LE TRAITEMENT DE L'ENDOMÉTRIOSE

(30) Priority: 21.03.2016 IT UA20161865
(43) Date of publication of application: 27.09.2017
(73) Proprietor: Napoletano, Ilaria, 04100 Latina (LT) (IT)
(72) Inventor: NAPOLETANO, Ilaria, 04100 Latina LT (IT); LANDI, Lapo, 50040 Calenzano FI (IT)
(74) Representative: Perani & Partners S.p.A.

(56) References cited:
- CN-A- 1 762 236
- JP-A- 2004 196 761
- JP-A- 2005 263 679
- Solomon P Wasser: "Shiitake (Lentinus edodes) INTRODUCTION", Encyclopedia of dietary supplements , 1 December 2005 (2005-12-01), XP055305769, DOI: 10.1081/E-EDS-120024880 Retrieved from the Internet: URL:http://www.dogcancer.net/wp-content/up loads/2014/11/shiitake.pdf [retrieved on 2016-09-27]
- Anonymous: "Endometriosis", , 15 September 2015 (2015-09-15), XP055306440, Retrieved from the Internet: URL:http://www.herbs2000.com/disorders/end ometriosis.htm [retrieved on 2016-09-29]
- Anonymous: "Infertility and Endometriosis", , 6 April 2015 (2015-04-06), XP055306441, Retrieved from the Internet: URL:http://www.sisterzeus.com/inferendo.ht ml [retrieved on 2016-09-29]

## Description

### FIELD OF THE INVENTION

The present invention relates to the use of lentinan or a shiitake extract containing said active ingredient for the treatment and prevention of endometriosis.

### PRIOR ART

Endometriosis is a still poorly known disease that affects women of childbearing age: it is estimated that only in Italy, 3 million women are affected, even 1 out of 2 in the age group ranging from 29 to 39 years. The disease is caused by certain cells of the uterine mucous membrane that are implanted in regions other than female uterus: the outer wall of the uterus, ovaries, fallopian tubes, peritoneum, vagina, bowel, bladder and more rarely in other organs such as the lungs and skin. One of the main problems with endometriosis is the difficulty of diagnosis: much often or even too often, the correct diagnosis is only achieved after countless cycles of therapy directed at the treatment of other types of diseases. In fact, the growing pains associated with the menstrual cycle are considered physiological and therapeutic insights are postponed.

Endometriosis cannot be permanently cured, but a successful control of symptoms can be achieved: the first supportive treatment is often to recourse to the use of the contraceptive pill, which prevents ovulation, thereby reducing the endometrial growth and simultaneously the pain associated with the cycle. In some cases, the pill is prescribed continuously, i.e. eliminating the 7 days suspension. Alternatively, other hormonal drugs may be prescribed with the same objective. Recourse to surgery through laparoscopy or through traditional surgery is generally followed when the drugs taken orally are not sufficient, but also in this case it may not be a definitive solution. The latest report on therapeutic efficacy reported by Cochrane ("Endometriosis an overview of Cochrane reviews Julie Brown et al., 10 March 2014"), identifies the most effective therapy for women of childbearing age, the hormone therapy based on gonadotropin-releasing hormone analogues (GnRH), the intrauterine release system of levonorgestrel (LNG-IUD), danazol and finally the administration of a progestin and anti-progestin. Since the endometriotic foci are triggered by the female sex hormones (estrogens and progestins), drug treatments are directed to regulating or suppressing the effect of these hormones. This is accomplished, for instance, with the intake of corpus luteum hormones (progestins). If a complete suppression of the ovarian function is necessary, the estrogen formation is prevented by a treatment with the so-called GnRH agonists. The administration of GnRH drains the endometriotic foci, thereby obtaining pain relief and preventing the formation of new endometriotic foci. However, infertility produced by hormone therapies based on progesterone and not always encouraging results obtained by laparoscopy have led to test the efficacy of alternative remedies, for example with Chinese herbs. While these remedies are natural and non-invasive, they do not seem to gain a decisive or replacement role with respect to other therapies which, nevertheless, do not solve the effects of post-pregnancy endometriosis.

The medicinal qualities of mushrooms have been known for thousands years and their consumption is an old tradition, especially in China and Japan. Mushrooms are also known to be functional foods due to the presence of bioactive compounds that often provide benefits to human health.

One of these powerful bioactive compounds is β-glucan, which comprises a backbone of glucoside repeating units bound by (1→3)-glycosidic bonds with attached branched points β-(1→6) showing antitumor properties. The bioactive substances of this type available on the market, such as schizophyllan, lentinan, grifolano, PSP (polysaccharide-peptide complex) and PSK (polysaccharide-protein complex) showed evident clinical results. The immunomodulating activity of fungal polysaccharides is to stimulate natural killer cells, in other words the immune system responses mediated by T cells, B cells, neutrophils and macrophages mediated by such as particular types of receptors such as toll-like, lactosylceramides, etc. The β-glucans depending on the particular structure have affinity for these particular receptors and are therefore able to trigger specific responses by the host against specific tumours (Antitumor activity of mushroom polysaccharides: a review. L. Rent et al.Food funct. 2012 Nov 3; (11):1118-30. doi: 10.1039/c2fo10279j.). In particular lentinan, characterized by the following formula: 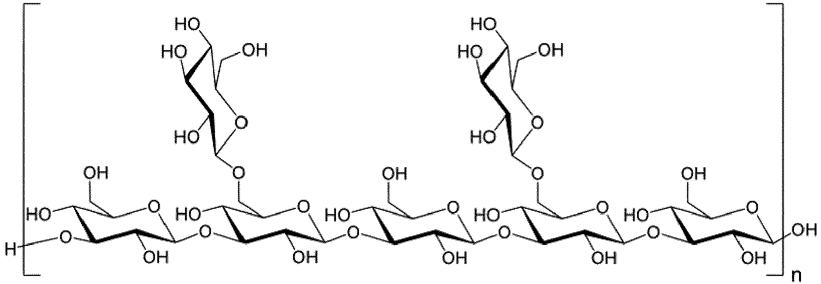 is one of the active ingredients present in larger amounts in shiitake (*Lentinula edodes).* In its pure form obtained by isolation from said mushroom, it was approved by the Ministry of Health in Japan as adjuvant combined with a chemotherapeutic agent of a conventional type, as it modulates the immune response in particular in the treatment of gastric cancer (Oba et al., "Individual patient based meta-analysis of lentinan for unresectable/recurrent gastric cancer" 2009 Anticancer Res 29(7):2739-45.; Ina et al. "The use of lentinan for treating gastric cancer." Anticancer Agents Med. Chem 2013 Jun; 13(5): 681-8)". Lentinan also seems to possess antiangiogenic activity, although Shiitake contains another organosulfur compound, lenthionine, which seems to share the antiangiogenic activity with lentinan. Custer C. Deocaris, Ma. Consolacion P. de Castro, Angel T. Oabel, Elisa L. Co and Elmer-Rico E. Mojica, 2005. Screening for Anti-angiogenic Activity in Shiitake Mushroom (Lentinus edodes Berk) Extracts. Journal of Medical Sciences, 5: 43-46.

### SUMMARY OF THE INVENTION

The Applicant has now found that lentinan can be used effectively for the treatment and prevention of endometriosis.

The Applicant has in fact found that lentinan exerts a cytotoxic and proapoptotic effect against endometrial cells of patients affected by endometriosis.

Moreover, due to the antiangiogenic activity, lentinan prevents the engraftment of endometrial cells outside the womb.

### DETAILED DESCRIPTION OF THE INVENTION

Preferably, lentinan is administered for use according to the present invention in the form of a Shiitake extract by systemic or topical route, in daily doses ranging between 10 and 1000 mg.

The Shiitake extract for the purposes of the present invention contains between 1.5 and 25% by weight, and preferably between 10 and 20% by weight of lentinan.

The use of shiitake instead of lentinan alone may be advantageous because, as mentioned above, it contains further active ingredients which along with lentinan may contribute to the effectiveness of treatment, such as the lenthionine which, as specified above, has also anti-angiogenesis activity.

Preferably, shiitake is administered together with an extract of another mushroom, reishi (*Ganoderma lucidum*), more preferably in daily doses ranging from 10 and 1000 mg per day.

Shiitake for use according to the present invention, optionally associated with reishi, can be optionally administered along with agnus castus (*Vitex Agnocastus).* The agnus castus extract, by virtue of the sedative and antispasmodic properties thereof, helps to relieve pelvic pain caused by endometriosis.

Agnus castus optionally administered in combination with shiitake and optionally with reishi is administered in daily doses of between 10 and 200 mg, preferably 100 mg daily.

More preferably, for use according to the present invention, it is provided that shiitake optionally in association with reishi and/or agnus castus is administered in combination with at least one active ingredient selected from at least one of the following classes consisting of antioxidants, vitamins and hormones.

Preferably, these further active ingredients are selected from: Vitamin A, a vitamin of the B group such as vitamin B1, B6, B9 (folic acid) vitamin B12, vitamin C, vitamin D, vitamin E, α-lipoic acid, Resveratrol, monacolin and in particular monacolin K from red yeast rice (Monascus Purpureus), N-acetylcysteine, eicosapentaenoic acid (EPA), docosaesaenoic acid (DHA), a hormone normally employed for the treatment of endometriosis, for example gonadotropin-releasing hormones (GnRH), levonorgestrel, danazol, progestins and antiprogestins.

Shiitake optionally administered in combination with reishi and agnus castus, and at least one of the above active ingredients with antioxidant and/or anti-inflammatory activity, vitamins and/or hormones in patients with chronic endometriosis, these show a marked clinical improvement, which not only result in a reduction of pelvic pain, but also reduces renal dilatation induced by endometriosis with increased fluidity of the menstrual cycle, which also becomes painless. For use according to the present invention in a particularly preferred embodiment of the invention, lentinan or the Shiitake extract optionally associated with reishi and/or agnus castus is administered with at least one of the following active ingredients: α-lipoic acid in daily doses ranging between 100 and 200 mg, monacolin in doses of no less than 10 mg, even more preferably equal to 10 mg, N-acetyl-cysteine in doses of no less than 600 mg, even more preferably 600 mg, eicosapentaenoic acid (EPA) between 200 and 500 mg, even more preferably between 228 and 456 mg, docosahexaenoic acid (DHA) between 150 and 300 mg, vitamin D between 1 and 20 µg, even more preferably 5-20 µg, folic acid in an amount of no less than 400 µg, preferably 400 µg.

For use according to the present invention, shiitake and any further active ingredients can be administered by systemic or topical route, either separately or in a single formulation, i that can be suitable for systemic administration or for topical administration.

The preferred systemic routes of administration are oral and parenteral, preferably intramuscular injection administration or intravenous injection administration. Particularly preferred for use according to the present invention is oral administration.

Preferred topical routes are vaginal and anal. Vaginal administration is particularly preferred.

The formulations for use according to the present invention preferably contain a shiitake extract containing said lentinan in combination with suitable excipients and/or diluents.

Shiitake contains lentinan at the above %w/w concentrations, i.e. 1.5-25%, more preferably 10-20%. Shiitake for the use according to the present invention is administered in formulations preferably also containing reishi and/or agnus castus along, in combination with suitable excipients and/or diluents.

More preferably, in addition to shiitake, optionally with reishi and/or agnus castus, These formulations contain at least one of the above active ingredients selected from at least one of the classes consisting of: anti-inflammatory, antioxidants, vitamins and/or hormones.

When Shiitake is administered for the use according to the present invention by oral route , it is administered in the form of tablets, capsules, more preferably capsules with a vegetable gelatin enclosure, single-dose granular sachets that can be administered directly in the mouth or dispersed in water before administration, liquid or semi-liquid beverages.

When Shiitake fpr the use according to the invention is administered by parenteral route preferably it is administered by intramuscular or intravenous injection in the form of vials in which shiitake and any other active ingredients are dispersible in saline, contained into another container, where for the purposes of the present invention, saline means an aqueous solution containing 0.9% W/V of NaCl (i.e. about 9 g/L).

According to the present description, vaginal topical formulations mean both actual vaginal formulations and those in the form of capsules and tablets, creams, ointments, douches, or devices that are inserted by medical personnel through the vagina inside the uterus. These devices, also referred to by the acronym IUD, are capable of releasing shiitake for the use according to the present invention and any additional active ingredients over a specific period of time. After this time, the device now devoid of active ingredients can be removed from the uterus, always by medical personnel.

Preferred vaginal formulations for the use according to the present invention are tablets, capsules, pessaries, douches. Anal topical formulations are instead preferably in the form of creams, capsules, suppositories, ointments, foams, intestinal lavages or mini-enemas.

Shiitake extracts and optionally reishi and/or agnus castus are preferably in the form of freeze-dried micronized powders so as to allow their dispersibility in physiological solutions, in topical or injection vaginal preparations, in particular for making douches. The same extracts are preferably in granular form prepared with conventional granulation processes, in particular for the preparation of tablets that, depending on the excipients, can be administered orally or vaginally. As regards the oral or injection systemic formulations for the use of Shitake according to the present invention , they can be for purely pharmaceutical use, in this case they are also preferably associated with hormones. However, the same type of oral formulations may also be used as dietary supplements, nutraceuticals or medical devices.

As regards vaginal topical formulations, for the use of Shitake according to the present invention are preferably in the form of tablets, capsules, douches, pessaries, as well as anal topical formulations, they may be marketed as medical devices.

By way of illustrative non-limiting purpose, for the use of Shitake according to the present invention an oral formulation may contain as active ingredients:
- 500 mg shiitake titrated at 10% in lentinan
- 350 mg reishi
- 10 µg vitamin D

## Claims

1. Shiitake extract for use in the treatment and prevention of endometriosis, wherein said Shiitake extract contains between 1,5% and 25% by weight of Lentinan, and said shiitake extract is administered in daily doses comprised between 10 and 1000 mg.

2. Shiitake extract for use according to claim 1, wherein said shiitake extract is administered in association with reishi preferably in daily doses comprised between 10 and 1000 mg.

3. Shiitake extract for use according to any one of claims 1-2, wherein said shiitake extract is administered in association with agnus castus, preferably in daily doses comprised between 10 and 200 mg.

4. Shiitake extract for use according to any one of claims 1-3, wherein said shiitake is administered in association with at least one active ingredient selected in at least one of the following classes consisting of antioxidants, anti-inflammatories, vitamins and hormones.

5. Shiitake extract for use according to claim 4, wherein shiitake extract is administered in association with at least one of the following active ingredients selected from: Vitamin A, a vitamin of the B group, selected from vitamin B1, B6, B9 (folic acid) vitamin B12, vitamin C, vitamin D, vitamin E, α-lipoic acid, Resveratrol, monacolin, N-acetylcysteine, eicosapentaenoic acid (EPA), docosaesaenoic acid (DHA), a hormone normally employed for the treatment of endometriosis, selected from gonadotropin-releasing hormones (GnRH), levonorgestrel, danazol, progestins and antiprogestins.

6. Shiitake extract for use according to claim 5, wherein shiitake extract is administered in association with at least one of the following active ingredients selected from:
• α-lipoic acid preferably in daily doses comprised between 100 and 200 mg,
• Monacolin preferably in daily doses not lower than 10 mg,
• N-acetylcysteine, preferably in daily doses not lower than 600mg,
• eicosapentaenoic acid (EPA) preferably in daily doses comprised between 200 and 500 mg,
• docosaesaenoic acid (DHA), preferably in daily doses comprised between 150 and 300 mg,
• folic acid, preferably in daily doses not lower than 400µg and
• vitamin D, preferably in daily doses comprised between 1 and 20µg.

7. Shiitake extract for use according to any one of claims 1-6, wherein said shiitake extract is administered by systemic oral route or by parenteral route selected from intramuscular injection and intravenous injection.

8. Shiitake extract for use according to any one of claims 1-6 wherein said lentinan or shiitake extract is administered by topical route, selected from topical-vaginal and topical-anal route.

9. Shiitake extract for use according to any one of claims 1-7, wherein when said shiitake extract is administered by systemic oral route in the form of: tablets, capsules, more preferably capsules, single-dose granular sachets that can be administered directly in the mouth or dispersed in water before administration, liquid or semi-liquid beverages.

10. Shiitake extract for use according to any one of claims 1-7, wherein when said Shiitake is administered by parenteral administration, it is administered by intramuscular or intravenous injection in the form of vials wherein the shiitake extract and any other active ingredients are in freeze-dried form easily dispersible in physiological solution.

11. Shiitake extract for use according to any one of claims 1-6 and 8, wherein when said Shitake is administered by topical vaginal route it is in the form of tablets, capsules, douches, pessaries, intrauterine devices capable of releasing shiitake and any other active ingredient, or when it is administered by topical anal route it is in the form of tablets, capsules, lotions, ointments, creams, foams, suppositories or intestinal lavages, mini-enemas.

## Patentansprüche

1. Shiitake-Extrakt zur verwendung in Behandlung und Vorbeugung von Endometriose, worin die Konzentration des Shiitake-Extrakts zwischen 1,5 und 25% Lentinansgewichts liegt und Shiitake-Extrakt in täglichen Dosen zwischen 10 und 1000 mg verabreicht wird.

2. Shiitake-Extrakt zur Verwendung nach Anspruch 1, wobei der Shiitake-Extrakt mit Reishi in täglichen Dosen zwischen 10 und 1000 mg verabreicht wird.

3. Shiitake-Extrakt zur Verwendung nach Ansprüche 1-2, wobei der Shiitake-Extrakt mit Agnus Castus vorzugsweise in täglichen Dosen zwischen 10 und 200mg verabreicht wird.

4. Shiitake-Extrakt zur Verwendung nach Ansprüche 1-3, wobei der Shiitake-Extrakt mit mindestens ein Wirkstoff zwischen Antioxidantien, Antiphlogistikum, Vitamine und Hormone ausgewählt wird.

5. Shiitake-Extrakt zur Verwendung nach Anspruch 4, wobei der Shiitake-Extrakt mit mindestens eins folgenderen Wirkstoffe ausgewählt wird,: Vitamin A, ein Vitamin der B-Gruppe, wie Vitamin B1, Vitamin B6, Vitamin B9 (Folsäure), Vitamin B12, Vitamin C, Vitamin D, Vitamin E, Alpha-Liponsäure, Resveratrol, Monacolin, N-Acetylcystein, Eicosapentaensäure (EPA), Docosaensäure (DHA), ein Hormon, das normalerweise zur Behandlung von Endometriose verwendet wird, und das zwischen Gonadotropin-loslassende Hormone (GnRH) ausgewählt wird, wie Levonogestrel, Danazol, Progesteron und Antiprogesteron.

6. Shiitake-Extrakt zur Verwendung nach Anspruch 5, wobei Shiitake-Extrakt mit mindestens eins folgenderen Wirkstoffe ausgewählt wird,:
• Alpha-Liponsäure, vorzugsweise in täglichen Dosen zwischen 100 und 200mg,
• Monacolin, vorzugsweise in täglichen Dosen nicht weniger als 10mg,
• N-Acetylcystein, vorzugsweise in täglichen Dosen nicht weniger als 600mg,
• Eicosapentaensäure (EPA), vorzugsweise in täglichen Dosen zwischen 200 und 500mg,
• Docosaensäure (DHA), vorzugsweise in täglichen Dosen zwischen 150 und 300mg,
• Folsäure, vorzugsweise in täglichen Dosen nicht weniger als 400µg,
• Vitamin D, vorzugsweise in täglichen Dosen zwischen 1 und 20µg.

7. Shiitake-Extrakt zur Verwendung nach Ansprüche 1-6, wobei Shiitake-Extrakt bei systemische-orale oder parenterale Route verabreicht wird, wie intramuskuläre Injektion oder intravenöse Injektion.

8. Shiitake-Extrakt zur Verwendung nach Ansprüche 1-6, wobei Lentinan oder Shiitake-Extrakt topisch bei Vagina und bei Rektum verabreicht wird.

9. Shiitake-Extrakt zur Verwendung nach Ansprüche 1-7, wobei, Shiitake-Extrakt systemisch-oral verabreicht wird durch Tabletten, Kapseln, vorzugsweise Kapseln, Einzeldosis-Granulat beutel direkt in der Mund oder in Wasser dispergiert, in flüssigen oder halbflüssigen Getränken.

10. Shiitake-Extrakt zur Verwendung nach Ansprüche 1-7, wobei Shiitake-Extrakt bei parenterale Route verabreicht wird, durch intramuskuläre Injektion oder intravenöse Injektion durch Fläschchen, worin der Shiitake-Extrakt oder andere Wirkstoffe lyophilisiert sind, leicht in physiologischer Lösung dispergierbar sind.

11. Shiitake-Extrakt zur Verabreichnung nach Ansprüche 1-6 und 8, wobei Shiitake-Extrakt bei der topische vaginale Route verabreicht wird, die ist durch Tabletten, Kapseln, Duschen, Pessare, Intrauterinpessare für die Freisetzung des Shiitake-Extrakt oder der anderen Wirkstoffe, oder, wenn die Verabreichnung ist bei der topische anale Route, die ist durch Tabletten, Kapseln, Lotionen, Salben, Cremes, Schäume, Zäpfchen oder Darmspülungen, Mini-Einläufe.

## Revendications

1. L'extrait de Shiitake pour utilisation dans le traitement et la prévention de l'endométriose, à une concentration de l'extrait de Shiitake entre 1,5% et 25% du poids du lentinane, et l'extrait de Shiitake est administré en doses quotidiennes entre 10 et 1000mg.

2. L'extrait de Shiitake pour l'utilisation dans la revendication 1, où l'extrait de Shiitake est administré en association avec Reishi en doses quotidiennes entre 10 et 1000mg.

3. L'extrait de Shiitake pour l'utilisation selon les revendications 1-2, où l'extrait de Shiitake est administré en association avec agnus castus, préférablement en doses quotidiennes entre 10 et 200mg.

4. L'extrait de Shiitake pour l'utilisation selon les revendications 1-3, où Shiitake est administré en association avec au moins une parmi les suivantes substances actives sélectionnées, dont les suivantes catégories de antioxydants, anti-inflammatoires, vitamines et hormones.

5. L'extrait de Shiitake pour l'utilisation selon revendication 4, où l'extrait Shiitake est administré en association avec au moins une dell suivantes substances actives sélectionnées parmi: Vitamine A, une vitamine du groupe B, dont vitamine B1, B6, B9 (acide folique), Vitamine B12, Vitamine C, Vitamine D, Vitamine E, Acide alpha-lipoĩque, Resvératrol, Monacoline, N-acétyl-cystéine, Acide Eicosapentaénoïque (EPA), Acide docosahexaénoïque (DHA), un hormone normalement utilisé dans le traitment de l'endométriose, sélectionné parmi les hormones qui relâchent gonadotrophine (GnRH), lévonogestrel, danazol, progestatifs et antiprogestatifs.

6. L'extrait de Shiitake pour l'utilisation selon la revendication 5, où l'extrait de Shiitake est administré en association avec au moins une parmi les suivantes substances actives sélectionnées:
• Acide alpha-lipoĩque, préférablement en doses quotidiennes entre 100 et 200mg,
• Monacoline, préférablement en doses quotidiennes pas en-dessous de 10mg,
• N-acétyl-cystéine, prérérablement en doses quotidiennes pas en-dessous de 600mg,
• Acide Eicosapentaénoïque (EPA),préférablemt en doses quotidiennes situées entre 200 et 500mg,
• Acide docosahexaénoïque (DHA), préférablemt en doses quotidiennes situées entre 150 et 300mg,
• Acide folique, préférablemtn en doses quotidiennes pas en-dessous de 400µg et
• Vitamine D, préférablement en doses quotidiennes situées entre 1 et 20µg.

7. L'extrait de Shiitake pour l'utilisation selon les revendications 1-6, où l'extrait de Shiitake est administré par voie systémique oral ou par voie parentérale, sélectionnée entre injection intramusculaire et injection intraveneuse.

8. L'extrait de Shiitake pour l'utilisation selon les revendications 1-6, où l'extrait de Lentinane ou l'extrait de Shiitake est administré par voie topique, sélectionnée entre voie topique vaginale et topique rectale.

9. L'extrait de Shiitake pour l'utilisation selon le revendications 1-7, ou l'extrait de Shiitake est administré par voie systémique orale sous forme de: tablettes, capsules, de préférence capsules, sachets granulaires à administration unique directement par bouche ou dissous dans l'eau, en boissons liquides ou semi-liquides.

10. L'extrait de Shiitake pour l'utilisation selon les revendications 1-7, ou l'extrait de Shiitake est administré par voie parentérale, par injection intramusculaire ou intraveneuse sous forme de flacons, si l'extrait de Shiitake ou les autres substances actives sont lyophylisés et dispersibles facilement en solution physiologique.

11. L'extrait de Shiitake pour l'utilisation déclarée dans toutes les revendications 1-6 et 8, où l'extrait de Shiitake est administré par voie vaginale sous forme de tablettes, capsules, douches, pessaires, dispositifs intra-utérins capables de relâcher le Shiitake ou toutes les autres substances actives, ou si il est administré par voie anale topique sous forme de tablettes, capsules, lotions, onguents, crèmes, mousses, suppositoires ou lavages intestinaux, mini-lavements.
